# EUROPEAN PATENT APPLICATION

(11) **EP 2 626 096 A1**
(43) Date of publication of application: **14.08.2013**
(21) Application number: 12155011.5
(22) Date of filing: 10.02.2012
(51) Int. Cl.: A61M 5/24

(54) **Medicament delivery device with needle assembly removal mechanism**

(71) Applicant: Sanofi-Aventis Deutschland GmbH, 65929 Frankfurt am Main (DE)
(72) Inventor: The designation of the inventor has not yet been filed

(57) **Abstract**

Described is a medicament delivery device (100) comprising a housing (102) having a distal end adapted to engage a removable needle assembly (400), a dosing mechanism (300) coupled to the housing (102), and a sleeve (202) coupled to the housing (102) and having a distal end (206) with one or more projections (208). Movement of the dosing mechanism (300) causes axial translation of the sleeve (202) relative to the housing (102) and causes the one or more projections (208) to extend from a retracted position relative to the housing (102) beyond the distal end of the housing (102) to disengage the removable needle assembly (400) from the housing (102).

## Description

### Technical Field

The invention relates to a medicament delivery device with a needle assembly removal mechanism device adapted to remove a needle assembly from the medicament delivery device.

### Background of the Invention

Administering a medicament by injection is a process which presents a number of risks and challenges for patients and healthcare professionals, both mental and physical. Improper handling of medicament delivery devices may result in needle stick injuries. In any situation in which a patient or healthcare professional is required to manually attach/remove the needle assembly, there is a risk of needle stick injury.

Conventional needle assemblies are attached to medicament delivery devices by a threaded engagement. Thus, after an injection, either a patient or a physician/nurse will have to remove the needle assembly by unscrewing it. As long as the needle assembly must be touched, there exists the risk of a needle stick injury.

Thus, there is a need for a mechanism for safely removing a needle assembly from a medicament delivery device.

### Summary of the Invention

It is an object of the present invention to provide a medicament delivery device with a needle assembly removal mechanism for removing a needle assembly from the medicament delivery device.

In an exemplary embodiment of the present invention, a medicament delivery device comprises a housing having a distal end adapted to engage a removable needle assembly, a dosing mechanism coupled to the housing, and a sleeve coupled to the housing and having a distal end with one or more projections. Movement of the dosing mechanism causes axial translation of the sleeve relative to the housing and causes the one or more projections to extend from a retracted position relative to the housing beyond the distal end of the housing to disengage the removable needle assembly from the housing.

In an exemplary embodiment, the dosing mechanism includes a dose-setter that is rotatable and axially translatable relative to the housing. The dose-setter includes a proximal end with a flat or contoured surface.

In an exemplary embodiment, the sleeve includes a proximal end with a flat or contoured surface.

In an exemplary embodiment, the housing includes a groove adapted to mate with an axial spline on the sleeve. The dose-setter is telescopically coupled to the sleeve.

In an exemplary embodiment, the medicament delivery device further comprises a spring biasing the sleeve or the one or more projections toward the retracted position.

In an exemplary embodiment, the medicament delivery device further comprises a latching mechanism adapted to prevent movement of the sleeve relative to the housing until a dose has been delivered by the dosing mechanism. The latching mechanism includes a peg on the dose-setter adapted to mate with a channel on the sleeve, and the channel includes an abutment stop adapted to engage the peg. Axial movement of the dose-setter relative to the housing causes the peg to engage the abutment stop and causes simultaneous translation of the dose-setter and the sleeve relative to the housing.

In an exemplary embodiment, the distal end of the sleeve includes a shoulder adapted to engage a shoulder on the distal end of the housing.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: shows an exemplary embodiment of a medicament delivery device including an exemplary embodiment of a needle assembly removal mechanism according to the present invention before use,
- Figure 2: shows an exemplary embodiment of a medicament delivery device including an exemplary embodiment of a needle assembly removal mechanism according to the present invention during medicament delivery,
- Figure 3: shows an exemplary embodiment of a medicament delivery device including an exemplary embodiment of a needle assembly removal mechanism according to the present invention after medicament delivery, and
- Figure 4: shows a sectional view of an exemplary embodiment of a medicament delivery device including an exemplary embodiment of a needle assembly removal mechanism according to the present invention after medicament delivery.

Corresponding parts are marked with the same reference symbols in all figures.

### Detailed Description

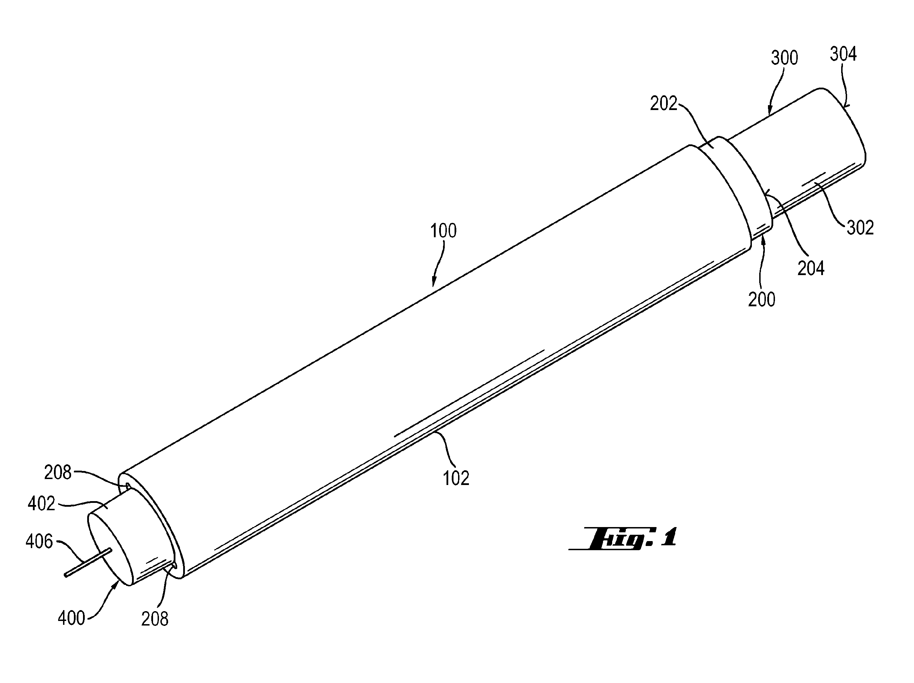

Figure 1 shows an exemplary embodiment of a medicament delivery device 100 including an exemplary embodiment of a needle assembly removal mechanism 200 according to the present invention before use. The delivery device 100 may include, but is not limited to, an autoinjector, a pen injector, a syringe, a safety syringe, etc.

In an exemplary embodiment, the delivery device 100 comprises a housing 102 which encases a cartridge (not shown) of a medicament. An dosing mechanism 300 may be coupled to a proximal end of the housing 102. In an exemplary embodiment, the dosing mechanism 300 includes a dose-setter 302 which is rotatable relative to the housing 102 to position a stopper relative to the cartridge corresponding to a dose of the medicament that is intended to be delivered. As the dose-setter 302 is rotated relative to the housing 102, the dose-setter 302 may move axially relative to the housing 102. For example, rotation of the dose-setter 302 in a first angular direction may result in the dose-setter 302 moving axially in a proximal direction relative to the housing 102, while rotation of the dose-setter 302 in a second angular direction may result in the dose-setter 302 moving axially in a distal direction relative to the housing 102. While the exemplary embodiment describes the dose-setter 302 as being rotatable in two different angular directions relative to the housing 102, those of skill in the art will understand that the dose-setter 302 may be limited to rotation in one angular direction in other exemplary embodiments of the present invention. The dose-setter 302 may include a flat or contoured proximal end 304 which facilitates pushing the dose-setter 302 in a distal direction relative to the housing 102 to deliver the medicament in the cartridge.

In an exemplary embodiment, the needle assembly removal mechanism 200 includes a sleeve 202 slidably disposed in the housing 102. The dosing mechanism 300 may be telescopically arranged relative to the needle assembly removal mechanism 200. For example, the dose-setter 302 may be formed as a cylindrical sleeve which telescopes from the sleeve 202 of the needle assembly removal mechanism 200. In an exemplary embodiment, rotation and/or axial movement of the dose-setter 302 does not impart any rotational and/or axial movement to the sleeve 202. In an exemplary embodiment, the sleeve 202 is limited to axial movement relative to the housing 102 and does not rotate relative to the housing 102. For example, the sleeve 202 may include an axial spline (not shown) which engages a groove (not shown) formed on the housing 102 to prevent rotation of the sleeve 202 relative to the housing 102.

A proximal end 204 of the sleeve 202 may include a flat or contoured rim which is adapted to be co-planar with the proximal end 304 of the dose-setter 302 after the dose-setter 304 has moved axially relative to the housing 102 in the distal direction to deliver the dose of the medicament. For example, in an exemplary embodiment in which the proximal end 304 of the dose-setter 302 is concave, the rim of the proximal end 204 of the sleeve 202 may be angled to continue a curve of the concave surface of the proximal end 304 of the dose-setter 302.

A distal end 206 of the sleeve 202 may include one or more projections 208 which extend through one or more corresponding holes or slots formed in a distal end of the housing 102. The distal end of the housing 102 may have an engagement portion adapted to engage a needle assembly 400. In an exemplary embodiment, the needle assembly 400 is friction fit or snap-fit onto the engagement portion of the housing 102. When the needle assembly 400 is engaged to the engagement portion, the projections 208 may be in a retracted position (as shown in Figure 1), abutting or substantially in plane with a hub 402 of the needle assembly 400.

In an exemplary embodiment, the projections 208 and/or the sleeve 202 may be biased relative to the housing 102 by a spring (not shown). For example, the projections 208 may be biased in the retracted position so that they do not obstruct attempts to engage a needle assembly 400 to the engagement portion of the housing 102.

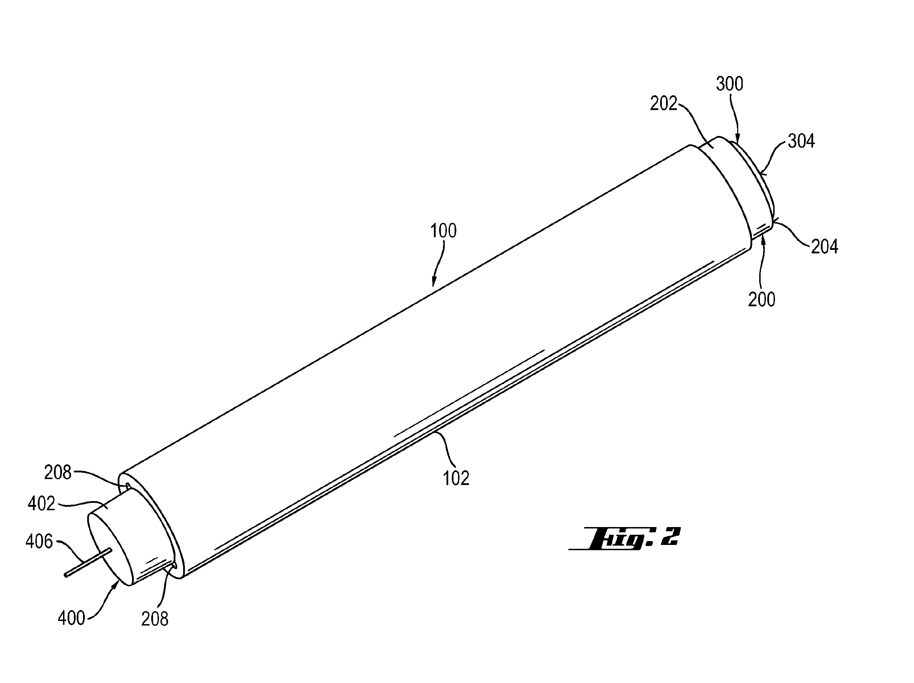

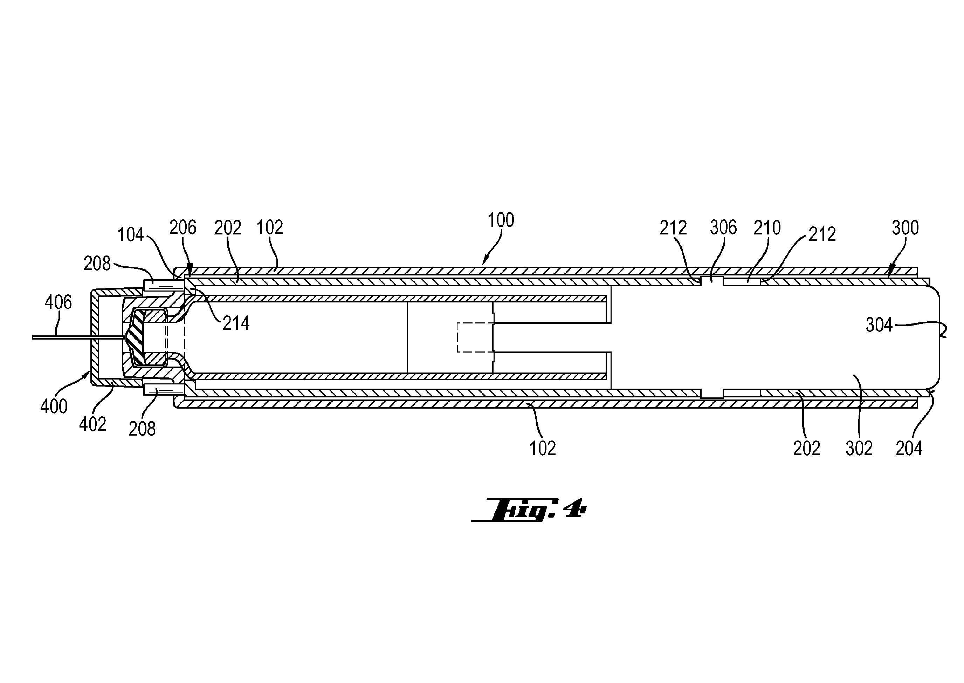

Figure 2 shows an exemplary embodiment of the delivery device 100 during medicament delivery. After the dose is set using the dosing mechanism 300, the dose-setter 302 is advanced axially in the distal direction relative to the housing 102, moving the stopper in the cartridge to dispense the medicament from a needle 406 on the needle assembly 400. Figure 2 shows a position the dose-setter 302 after the dose has been delivered. The proximal end 304 of the dose-setter 302 is substantially co-planar with the proximal end 204 of the sleeve 202. In an exemplary embodiment, a latching mechanism (shown in Figure 4) prevents the sleeve 202 from axial movement while the dose-setter 302 is being advanced to deliver the dose. For example, the dose-setter 302 may have an axial spline or peg 306 which mates with a groove or channel 210 on the sleeve 202 (those of skill in the art will understand that this arrangement may be reversed). The groove/channel 210 may have one or more abutment stops 212 adapted to transfer force to the spline/peg 306. During dose delivery, the spline/peg 306 may freely translate in the groove/channel 210.

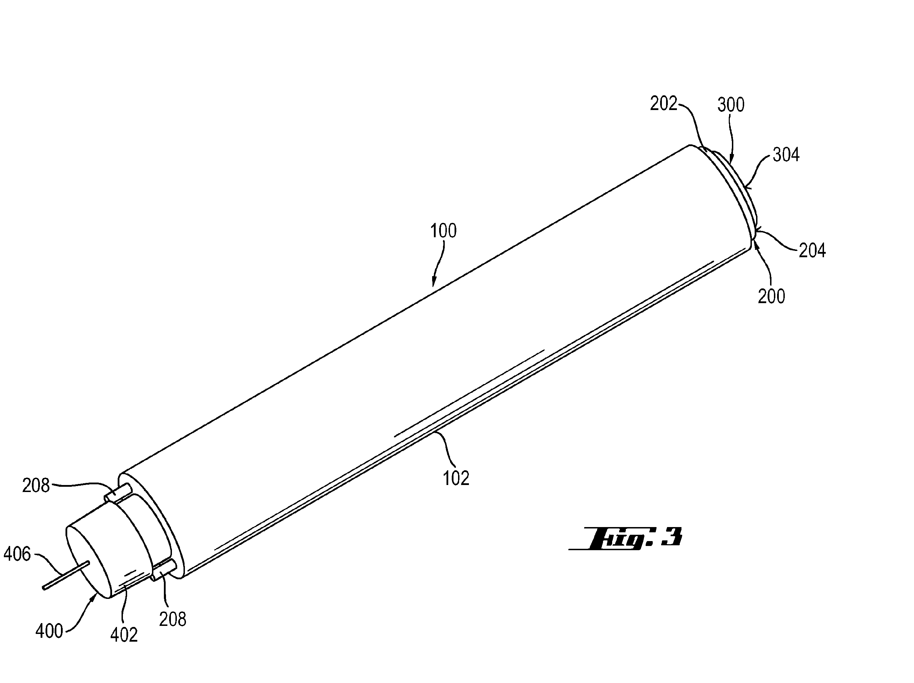

Figure 3 shows an exemplary embodiment of the delivery device 100 after medicament delivery. After the dose has been delivered, further advancement of the dose-setter 302 may engage the latching mechanism, causing the dose-setter 302 and the sleeve 202 to translate axially relative to the housing 102 in the distal direction. As the sleeve 202 translates distally, the projections 208 extend in the distal direction, disengaging the needle assembly 400 from the housing 102. In an exemplary embodiment, the projections 208 push the needle assembly 400 off of the engagement portion of the housing 102 (e.g., to deposit the used needle assembly in a sharps bin). In another exemplary embodiment, the projections 208 include a surface geometry adapted to disengage snaps or latches on the needle hub 402 from the engagement portion of the housing 102. For example, the snaps or latches may be resiliently formed in the needle hub 402, and the projections 208 may include angled surfaces to radially displace the snaps/latches. Further distal movement of the projections 208 may push the used needle assembly 400 off the housing 102.

Figure 4 shows a sectional view of the delivery device 100 after medicament delivery. As shown in the exemplary embodiment in Figure 4, after the dose is delivered and the proximal end 304 of the dose-setter 302 is substantially co-planar with the proximal end 204 of the sleeve 202, further advancement of the dose-setter 302 in the distal direction relative to the sleeve 202 causes the spline/peg 306 to abut a distal abutment stop 212 and push the sleeve 202 in the distal direction. The dose-setter 302 and the sleeve 202 may then translate axially in the distal direction together until shoulders 214 on a distal end of the sleeve 202 abut shoulders 104 on the distal end of the housing 102.

In an exemplary embodiment, as described above, translation of the sleeve 202 in the distal direction relative to the housing 102 may be against a biasing force of a spring (not shown) which biases the sleeve 202 and/or the projections 208 in the proximal direction. For example, the spring may be grounded against the shoulders 104 of the housing 102 and the shoulders 214 of the sleeve 202. When axial force is removed from the sleeve 202 and/or the dose-setter 302, the biasing force of the spring may return the projections 208 to the retracted position.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A medicament delivery device (100), comprising:
a housing (102) having a distal end adapted to engage a removable needle assembly (400);
a dosing mechanism (300) coupled to the housing (102);
a sleeve (202) coupled to the housing (102) and having a distal end (206) with one or more projections (208),
wherein, movement of the dosing mechanism (300) causes axial translation of the sleeve (202) relative to the housing (102) and causes the one or more projections (208) to extend from a retracted position relative to the housing (102) beyond the distal end of the housing (102) to disengage the removable needle assembly (400) from the housing (102).

2. The medicament delivery device (100) according to claim 1, wherein the dosing mechanism (300) includes a dose-setter (302) that is rotatable and axially translatable relative to the housing (102).

3. The medicament delivery device (100) according to claim 2, wherein the dose-setter (302) includes a proximal end (304) with a flat or contoured surface.

4. The medicament delivery device (100) according to any of the preceding claims, wherein the sleeve (202) includes a proximal end (204) with a flat or contoured surface.

5. The medicament delivery device (100) according to any of the preceding claims, wherein the housing (102) includes a groove adapted to mate with an axial spline on the sleeve (202).

6. The medicament delivery device (100) according to any of claims 2-4, wherein the dose-setter (302) is telescopically coupled to the sleeve (202).

7. The medicament delivery device (100) according to any of the preceding claims, further comprising:
a spring biasing the sleeve (202) or the one or more projections (208) toward the retracted position.

8. The medicament delivery device (100) according to any of the preceding claims, further comprising:
a latching mechanism adapted to prevent movement of the sleeve (202) relative to the housing (102) until a dose has been delivered by the dosing mechanism (300).

9. The medicament delivery device (100) according to claims 2 and 8, wherein the latching mechanism includes a peg (306) on the dose-setter (302) adapted to mate with a channel (210) on the sleeve (202), and wherein the channel (210) includes an abutment stop (212) adapted to engage the peg (306).

10. The medicament delivery device (100) according to claim 9, wherein axial movement of the dose-setter (302) relative to the housing (102) causes the peg (306) to engage the abutment stop (212) and causes simultaneous translation of the dose-setter (302) and the sleeve (202) relative to the housing (102).

11. The medicament delivery device (100) according to any of the preceding claims, wherein the distal end (206) of the sleeve (202) includes a shoulder (214) adapted to engage a shoulder (104) on the distal end of the housing (102).
